# EUROPEAN PATENT APPLICATION

(11) **EP 4 722 244 A1**
(43) Date of publication of application: **08.04.2026**
(21) Application number: 23936678.4
(22) Date of filing: 10.05.2023
(51) Int. Cl.: C07K 16/28, A61P 25/28, A61K 39/00

(54) **ANTI-TLR2 ANTIBODY AND USE THEREOF**

(30) Priority: 09.05.2023 KR 20230060053
(71) Applicant: Neuramedy Co., Ltd., Seoul 04796 (KR)
(72) Inventor: HAM, Sang Woo, Seoul 04796 (KR); YANG, Woo Seung, Seoul 04796 (KR); SHIN, Ji Min, Seoul 04796 (KR); LEE, Won Jae, Seoul 04796 (KR); LEE, Jun Sung, Seoul 04796 (KR); LEE, Seung Jae, Seoul 04796 (KR)
(74) Representative: HGF
(86) International application number: PCT/KR2023/006350
(87) International publication number: WO 2024/232454

(57) **Abstract**

The present invention relates to a novel use of an anti-TLR2 antibody and specifically, to a use of an anti-TLR2 antibody for the prevention or treatment of multiple system atrophy (MSA). The anti-TLR2 antibody according to the present disclosure exhibits the effects of significantly reducing disease marker expression, decreasing inflammasomes, and promoting neurofibril recovery at both multiple system atrophy cellular and animal levels, and thus can be variously applied in the field of preventing, alleviating, and treating multiple system atrophy.

## Description

### [Technical Field]

The present disclosure relates to a novel use of an anti-TLR2 antibody and more particularly, to a use of an anti-TLR2 antibody for the prevention or treatment of multiple system atrophy.

### [Background Art]

As the human lifespan increases, the incidence of neurodegenerative diseases, including Parkinson's disease, amyotrophic lateral sclerosis, multiple sclerosis, Huntington's disease, Alzheimer's disease, multiple system atrophy, multi-infarct dementia, and disciform macular degeneration, is also increasing. Currently, patients suffering from neurodegenerative diseases are estimated as 24 million people worldwide. In addition, cerebrovascular disease as another form of neurodegenerative disease or other diseases caused by trauma or injury are on the rise every year.

According to statistics from the Health Insurance Review & Assessment Service, there were a total of 1,951 patients received medical treatment for multiple system atrophy in 2020. The patients have more than doubled in five years from 926 in 2016.

Among these, particularly, multiple system atrophy (MSA) is a rare progressive neurodegenerative disease and characterized to be shown by combining various symptoms. People with this disease have symptoms similar to Parkinson's disease, such as cerebellar ataxia and autonomic failure of the autonomic nervous system, such as heart rate, blood pressure, sweating, bowel movement, and bladder control. However, an exact cause of multiple system atrophy has not yet been identified. There is currently no therapeutic agent developed, and drugs, such as a levodopa/carbidopa combination known as Sinemet, which is used to treat patients with Parkinson's disease, are also prescribed by targeting only a symptom control.

### [Disclosure]

### [Technical Problem]

Accordingly, the present inventors confirmed an excellent effect of an anti-TLR2 antibody on treatment of multiple system atrophy symptoms while conducting research to develop a therapeutic agent for multiple system atrophy, and then completed the present disclosure.

Therefore, an object of the present disclosure is to provide a composition for preventing or treating multiple system atrophy, including an antibody or an antigen binding fragment thereof that specifically binds to Toll-Like Receptor 2 (TLR2).

Another object of the present disclosure is to provide a method for treating multiple system atrophy, including administering, to a subject, an effective amount of an antibody or an antigen binding fragment thereof that specifically binds to TLR2.

### [Technical Solution]

In order to achieve the object, an aspect of the present disclosure provides a pharmaceutical composition for preventing or treating multiple system atrophy, including an antibody or an antigen binding fragment thereof that specifically binds to TLR2, including a heavy chain variable region including a heavy chain CDR1 including an amino acid sequence represented by SEQ ID NO: 1, a heavy chain CDR2 including an amino acid sequence represented by SEQ ID NO: 2, and a heavy chain CDR3 including an amino acid sequence represented by SEQ ID NO: 3; and a light chain variable region including a light chain CDR1 including an amino acid sequence represented by SEQ ID NO: 4, a light chain CDR2 including an amino acid sequence represented by SEQ ID NO: 5, and a light chain CDR3 including an amino acid sequence represented by SEQ ID NO: 6.

In addition, another aspect of the present disclosure provides a veterinary composition for preventing or treating multiple system atrophy, including an antibody or an antigen binding fragment thereof that specifically binds to TLR2, including a heavy chain variable region including a heavy chain CDR1 including an amino acid sequence represented by SEQ ID NO: 1, a heavy chain CDR2 including an amino acid sequence represented by SEQ ID NO: 2, and a heavy chain CDR3 including an amino acid sequence represented by SEQ ID NO: 3; and a light chain variable region including a light chain CDR1 including an amino acid sequence represented by SEQ ID NO: 4, a light chain CDR2 including an amino acid sequence represented by SEQ ID NO: 5, and a light chain CDR3 including an amino acid sequence represented by SEQ ID NO: 6.

In addition, yet another aspect of the present disclosure provides a method for treating multiple system atrophy, including administering, to a subject, an effective amount of an antibody or an antigen binding fragment thereof that specifically binds to TLR2, including a heavy chain variable region including a heavy chain CDR1 including an amino acid sequence represented by SEQ ID NO: 1, a heavy chain CDR2 including an amino acid sequence represented by SEQ ID NO: 2, and a heavy chain CDR3 including an amino acid sequence represented by SEQ ID NO: 3; and a light chain variable region including a light chain CDR1 including an amino acid sequence represented by SEQ ID NO: 4, a light chain CDR2 including an amino acid sequence represented by SEQ ID NO: 5, and a light chain CDR3 including an amino acid sequence represented by SEQ ID NO: 6.

### [Advantageous Effects]

According to the present disclosure, the anti-TLR2 antibody exhibits the effects of significantly reducing disease marker expression, decreasing inflammasomes, and promoting neurofibril recovery at both multiple system atrophy cellular and animal levels, and thus can be variously applied in the field of preventing, alleviating, and treating multiple system atrophy.

### [Description of Drawings]

FIG. 1 is a diagram showing results of confirming the characteristics of an in vitro multiple system atrophy cell model using human neural stem cells.
FIG. 2 is a diagram showing results of confirming DNA damage in an in vitro multiple system atrophy cell model using human neural stem cells.
FIG. 3 is a diagram showing results of confirming cellular senescence in an in vitro multiple system atrophy cell model using human neural stem cells.
FIG. 4 is a diagram showing results of confirming a GCI reduction effect according to NM-103 treatment in an in vitro multiple system atrophy cell model using human neural stem cells.
FIG. 5 is a diagram showing results of evaluating a DNA damage reduction effect according to NM-103 treatment in an in vitro multiple system atrophy cell model using human neural stem cells.
FIG. 6 is a diagram showing results of confirming a cellular senescence reduction effect according to NM-103 treatment in an in vitro multiple system atrophy cell model using human neural stem cells.
FIG. 7 is a diagram showing results of analyzing the survival rate of subjects according to NM-103 treatment in an in vivo multiple system atrophy model.
FIG. 8 is a diagram showing results of confirming a motor ability improvement effect according to NM-103 treatment in an in vivo multiple system atrophy model.
FIG. 9 is a diagram showing results of confirming a GCI reduction effect according to NM-103 treatment in an in vivo multiple system atrophy model.
FIG. 10A is a diagram showing results of confirming a neuroinflammation reduction effect according to NM-103 treatment in the Cortex of an in vivo multiple system atrophy model.
FIG. 10B is a diagram showing results of confirming a neuroinflammation reduction effect according to NM-103 treatment in the White matter of an in vivo multiple system atrophy model.
FIG. 10C is a diagram showing results of confirming a neuroinflammation reduction effect according to NM-103 treatment in the Hippocampus of an in vivo multiple system atrophy model.
FIG. 10D is a diagram showing results of confirming a neuroinflammation reduction effect according to NM-103 treatment in the Striatum of an in vivo multiple system atrophy model.
FIG. 10E is a diagram showing results of confirming a neuroinflammation reduction effect according to NM-103 treatment in the Substantia nigra of an in vivo multiple system atrophy model.
FIG. 11 is a diagram showing results of confirming an inflammasome reduction effect according to NM-103 treatment in an in vivo multiple system atrophy model.
FIG. 12 is a diagram showing results of confirming a neurofibril recovery effect according to NM-103 treatment in an in vivo multiple system atrophy model.

### [Best Mode]

Hereinafter, the present disclosure will be described in detail.

According to an aspect of the present disclosure, the present disclosure provides a composition for preventing or treating multiple system atrophy, including an antibody or an antigen binding fragment thereof that specifically binds to TLR2, including a heavy chain variable region including a heavy chain CDR1 including an amino acid sequence represented by SEQ ID NO: 1, a heavy chain CDR2 including an amino acid sequence represented by SEQ ID NO: 2, and a heavy chain CDR3 including an amino acid sequence represented by SEQ ID NO: 3; and a light chain variable region including a light chain CDR1 including an amino acid sequence represented by SEQ ID NO: 4, a light chain CDR2 including an amino acid sequence represented by SEQ ID NO: 5, and a light chain CDR3 including an amino acid sequence represented by SEQ ID NO: 6. The composition for preventing or treating multiple system atrophy according to the present disclosure may be a pharmaceutical composition or a veterinary composition.

As used in the present disclosure, the term "antibody" refers to an anti-TLR2 antibody that specifically binds to TLR2. The scope of the present disclosure includes not only a complete antibody form that specifically bind to TLR2, but also antigen binding fragments of the antibody molecule.

The complete antibody has a structure having two full-length light chains and two full-length heavy chains, and each light chain is linked to the heavy chain by disulfide bonds.

As used in the present disclosure, the term "heavy chain" refers to both a full-length heavy chain and a fragment thereof including a variable region domain VH including an amino acid sequence having a sufficient variable region sequence to impart specificity to the antigen and three constant region domains CH1, CH2 and CH3. In addition, as used in the present disclosure, the term "light chain" refers to both a full-length light chain and a fragment thereof including a variable region domain VL including an amino acid sequence having a sufficient variable region sequence to impart specificity to the antigen and a constant region domain CL.

The full-length antibody includes subtypes of IgA, IgD, IgE, IgM and IgG, and in particular, IgG includes IgG1, IgG2, IgG3 and IgG4. The heavy chain constant region has gamma (γ), mu (µ), alpha (α), delta (δ), and epsilon (ε) types, and has subclasses of gamma 1 (y1), gamma 2 (γ2), gamma 3 (y3), gamma 4 (γ4), alpha 1 (α1) and alpha 2 (α2). The light chain constant region has kappa (κ) and lambda (λ) types.

The antigen binding fragment of the antibody or the antibody fragment refers to a fragment having an antigen binding function, and includes Fab, F(ab'), F(ab')2, Fv, etc. Among the antibody fragments, the Fab has a structure having variable regions of the light and heavy chains, a constant region of the light chain, and a first constant region CH1 of the heavy chain, and has one antigen binding site. The Fab' is different from Fab by having a hinge-region containing one or more cysteine residues in a C-terminal of the heavy chain CH1 domain. The F(ab')2 is produced when the cysteine residues in the hinge-region of Fab' form a disulfide bond.

The Fv corresponds to the smallest antibody fragment having only a heavy chain variable region and a light chain variable region. A two-chain Fv has a heavy chain variable region and a light chain variable region which are linked by non-covalent bonds, and a single-chain Fv (scFv) may generally have the same dimer-like structure as the two-chain Fv because the heavy chain variable region and the light chain variable region are covalently linked by a peptide linker or directly linked at a C-terminal. These antibody fragments may be prepared using proteolytic enzymes (for example, Fab may be obtained by restriction cleavage of a complete antibody with papain, and F(ab')2 may be obtained by cleavage with pepsin), or by genetic recombination technology.

The "Fv" fragment is an antibody fragment that contains a complete antibody recognition and binding site. This region is a dimer in which one heavy chain variable domain and one light chain variable domain are linked.

The "Fab" fragment includes variable and constant domains of the light chain and a variable domain and a first constant domain (CH1) of the heavy chain. The F(ab')2 antibody fragment generally includes a pair of Fab' fragments covalently linked by cysteines in the hinge-region present at the C-terminal of the Fab' fragment.

The "single-chain Fv (scFv)" antibody fragment has a structure consisting of a single polypeptide chain including VH and VL domains of the antibody. A polypeptide linker may further be included between the VH and VL domains to allow the scFv to form a desired structure for antigen binding.

In one embodiment, the antibody of the present disclosure includes a monoclonal antibody, a multispecific antibody, a human antibody, a humanized antibody, a chimeric antibody, scFv, a Fab fragment, an F(ab')2 fragment, a disulfide-bond Fvs (sdFv), and an anti-idiotypic (anti-Id) antibody, or epitope-binding fragments of the antibodies, but is not limited thereto.

The heavy chain constant region may be selected from any one isotype of gamma (y), mu (µ), alpha (α), delta (δ), or epsilon (ε). For example, the constant region is gamma 1 (IgG1), gamma 2 (IgG2), gamma 3 (IgG3) or gamma 4 (IgG4). The light chain constant region may be a kappa or lambda type.

The monoclonal antibody refers to an antibody obtained from a substantially homogeneous antibody group, i.e., the same antibody except for possible naturally occurring mutations in which individual antibodies having the group may be present in a small amount. The monoclonal antibody is highly specific to be induced against a single antigenic site. In contrast to conventional (polyclonal) antibodies, which typically include different antibodies for different epitopes, each monoclonal antibody is indicated for a single epitope on the antigen.

The "epitope" means a protein determinant to which the antibody may specifically bind. The epitope usually consists of a chemically active surface molecule group, such as amino acids or sugar side chains, and generally has not only specific 3D structural characteristics, but also specific charge characteristics. Conformational and non-conformational epitopes are distinguished from each other in that the binding to the former is lost, but the binding to the latter is not lost in the presence of a denaturing solvent.

The "humanized" type non-human (e.g., mouse) antibody is a chimeric antibody that contains a minimal sequence derived from non-human immunoglobulin. In most cases, the humanized antibody is human immunoglobulin (recipient antibody) obtained by replacing residues from a hypervariable region of a recipient with residues from a hypervariable region of a non-human species (donor antibody), such as mouse, rat, rabbit, or non-human primate, which retains the desired specificity, affinity, and capacity.

The "human antibody" is a molecule derived from human immunoglobulin, and means that the entire amino acid sequence constituting an antibody, including complementarity determining regions and structural regions, is composed of human immunoglobulin.

The human antibody includes a fragment of the antibody exhibiting a desired biological activity as well as a "chimeric" antibody (immunoglobulin) in which some of the heavy and/or light chains are identical to or homologous to a corresponding sequence in an antibody derived from a specific species or belonging to a specific antibody class or subclass, while the remaining chain(s) are identical to or homologous to the corresponding sequence in an antibody derived from another species or belonging to another antibody class or subclass.

As used in the present disclosure, the "variable region" of the antibody refers to the light and heavy chain portions of an antibody molecule including amino acid sequences of complementarity determining regions (CDRs; that is, CDR1, CDR2, and CDR3) and a framework region (FR). VH refers to a variable domain of the heavy chain. VL refers to a variable domain of the light chain.

The "complementarity determining region (CDR)" refers to amino acid residues of an antibody variable domain, which is required for antigen binding. Each variable domain typically has three CDRs identified as CDR1, CDR2 and CDR3.

The "framework region (FR)" is variable domain residues other than the CDR residues. Each variable domain typically has four FRs of FR1, FR2, FR3 and FR4.

In a specific embodiment of the present disclosure, the antibody that specifically binds to TLR2 preferably includes a heavy chain variable region represented by an amino acid sequence represented by SEQ ID NO: 7.

In a specific embodiment of the present disclosure, the antibody that specifically binds to TLR2 preferably includes a light chain variable region represented by an amino acid sequence represented by SEQ ID NO: 8.

In a specific embodiment of the present disclosure, the antibody that specifically binds to TLR2 preferably includes a heavy chain represented by an amino acid sequence represented by SEQ ID NO: 9; and a light chain represented by an amino acid sequence represented by SEQ ID NO: 10.

The antibody or the antibody fragment of the present disclosure may include not only the sequence of the anti-TLR2 antibody of the present disclosure described herein, but also a biological equivalent thereof, within a range capable of specifically recognizing TLR2. For example, the amino acid sequence of the antibody may have additional changes to further improve the binding affinity and/or other biological properties of the antibody. These modifications include, for example, deletion, insertion and/or substitution of amino acid sequence residues of the antibody. These amino acid mutations are made based on the relative similarity of amino acid side-chain substituents, such as hydrophobicity, hydrophilicity, charges, sizes and the like. According to analysis of the size, shape and type of the amino acid side-chain substituent, it may be seen that arginine, lysine and histidine are all positively charged residues; alanine, glycine and serine have similar sizes; and phenylalanine, tryptophan and tyrosine have similar shapes. Accordingly, based on these considerations, arginine, lysine and histidine; alanine, glycine and serine; and phenylalanine, tryptophan and tyrosine may be biologically functional equivalents.

Considering the mutations having biological equivalent activity described above, the antibody of the present disclosure is interpreted to include sequences representing substantial identity with the sequence described in the sequence number. The substantial identity means a sequence exhibiting homology of at least 90%, most preferably homology of at least 95%, that is, homology of 96% or more, 97% or more, 98% or more, and 99% or more, when the sequence of the present disclosure is aligned to correspond to any other sequence as much as possible and the aligned sequence is analyzed using an algorithm commonly used in the art. Alignment methods for sequence comparison are known in the art. A NCBI Basic Local Alignment Search Tool (BLAST) is accessible from NBCI, etc., and may be used in conjunction with sequence analysis programs such as blastp, blasm, blastx, tblastn, and tblastx on the Internet. BLAST is accessible at www.ncbi.nlm.nih.gov/BLAST/. A sequence homology comparison method using these programs may be confirmed at www.ncbi.nlm.nih.gov/BLAST/blast_help.html.

Based thereon, the antibody or the antigen binding fragment thereof of the present disclosure may have homology of 90%, 91%, 92%, 93%, 94%, 95%, 96%, 97%, 98%, 99%, 99 % or more as compared with the specified sequence or the entire sequence described in the present disclosure. Such homology may be determined by sequence comparison and/or alignment by methods known in the art. For example, percent sequence homology of nucleic acids or proteins according to the present disclosure may be determined using a sequence comparison algorithm (i.e., BLAST or BLAST 2.0), manual alignment, or visual inspection.

In the present disclosure, the "prevention" refers to all actions that suppress clinical symptoms of a disease or delay the progression thereof by administering the composition according to the present disclosure, and the "treatment" refers to inhibition of development of clinical symptoms of the disease, and alleviation or elimination of clinical symptoms of the disease.

When the composition of the present disclosure is the pharmaceutical composition, the composition may further include a pharmaceutically acceptable carrier. The pharmaceutically acceptable carrier to be included in the composition of the present disclosure is generally used in preparation, and includes lactose, dextrose, sucrose, sorbitol, mannitol, starch, acacia gum, calcium phosphate, alginate, gelatin, calcium silicate, microcrystalline cellulose, polyvinylpyrrolidone, water, syrup, methylcellulose, methylhydroxybenzoate, propylhydroxybenzoate, talc, magnesium stearate, mineral oil, and the like, but is not limited thereto. The composition of the present disclosure may further include a lubricant, a wetting agent, a sweetening agent, a flavoring agent, an emulsifier, a suspension, a preservative, and the like, in addition to the ingredients.

The pharmaceutical composition of the present disclosure may be administered orally or parenterally, and in the case of parenteral administration, the pharmaceutical composition may be administered through intravenous injection, subcutaneous injection, intramuscular injection, intraperitoneal injection, intradermal administration, topical administration, intranasal administration, intrapulmonary administration, intrarectal administration, etc.

In the case of oral administration, since the proteins or peptides are digested, it is required to formulate the oral composition so as to coat an active agent or to be protected from decomposition at the stomach. In addition, the pharmaceutical composition may be administered by any device so that an active material is movable to a target cell.

A suitable dose of the composition according to the present disclosure varies depending on factors, such as a formulation method, an administration method, age, weight, sex, and medical condition of a patient, food, an administration time, an administration route, an excretion rate, and response sensitivity, and an ordinarily skilled physician may easily determine and prescribe a dose effective for desired treatment or prevention. For example, a daily dose of the pharmaceutical composition of the present disclosure is 0.0001 to 100 mg/kg. In the present disclosure, the term "pharmaceutically effective amount" means an amount sufficient to prevent or treat cancer or infectious diseases.

The pharmaceutical composition of the present disclosure may be formulated by using a pharmaceutically acceptable carrier and/or an excipient according to a method that may be easily performed by those skilled in the art to be prepared in a unit dose form or prepared by introduction into a multi-dose container. In this case, the formulations may also be forms of solutions, suspensions, or emulsions in oils or aqueous media or forms of extracts, powders, suppositories, powders, granules, tablets or capsules, and may further include a dispersant or a stabilizer.

When the composition of the present disclosure is the veterinary composition, the composition of the present disclosure may further include appropriate excipients and diluents commonly used in the preparation of the veterinary composition.

The excipients and diluents that may be included in the veterinary composition of the present disclosure may include lactose, dextrose, sucrose, sorbitol, mannitol, xylitol, erythritol, maltitol, starch, acacia gum, alginate, gelatin, calcium phosphate, calcium silicate, cellulose, methyl cellulose, microcrystalline cellulose, polyvinylpyrrolidone, water, methyl hydroxybenzoate, propyl hydroxybenzoate, talc, magnesium stearate, cetanol, stearyl alcohol, liquid paraffin, sorbitan monostearate, polysorbate 60, methylparaben, propylparaben, and mineral oils.

The veterinary composition of the present disclosure may further include fillers, anticoagulants, lubricants, wetting agents, flavorings, emulsifiers, preservatives, and the like. The veterinary composition of the present disclosure may be formulated by using methods known in the art so as to provide rapid, sustained, or delayed release of the active ingredient after administration to animals. The formulations may be forms of powders, granules, tablets, capsules, suspensions, emulsions, solutions, syrups, aerosols, soft or hard gelatin capsules, suppositories, sterile injectable solutions, sterile external preparations, etc. The veterinary composition of the present disclosure formulated above may be administered via various routes, including orally, transdermally, subcutaneously, intravenously, intrathecally, or intramuscularly.

The veterinary composition of the present disclosure may vary depending on the age, sex, and body weight of an animal, but may be administered once or several times a day in an amount of 0.1 to 100 mg/kg. In addition, the dose of β-NGF may be increased or decreased depending on a route of administration, severity of a disease, sex, body weight, age, etc. The dose does not limit the scope of the present disclosure in any aspect.

According to another aspect of the present disclosure, the present disclosure provides a method for treating multiple system atrophy, including administering, to a subject, an effective amount of an antibody or an antigen binding fragment thereof that specifically binds to TLR2, including a heavy chain variable region including a heavy chain CDR1 including an amino acid sequence represented by SEQ ID NO: 1, a heavy chain CDR2 including an amino acid sequence represented by SEQ ID NO: 2, and a heavy chain CDR3 including an amino acid sequence represented by SEQ ID NO: 3; and a light chain variable region including a light chain CDR1 including an amino acid sequence represented by SEQ ID NO: 4, a light chain CDR2 including an amino acid sequence represented by SEQ ID NO: 5, and a light chain CDR3 including an amino acid sequence represented by SEQ ID NO: 6.

In a specific embodiment of the present disclosure, the subject may be a subject expected to develop multiple system atrophy; a subject suffering from multiple system atrophy; or a subject completely cured of multiple system atrophy, but is not limited thereto.

Duplicated contents will be omitted in consideration of the complexity of the present specification, and terms not defined otherwise herein have the meanings commonly used in the art to which the present disclosure pertains.

### [Modes]

Hereinafter, the present disclosure will be described in more detail through Examples. These Examples are just illustrative of the present disclosure, and it will be apparent to those skilled in the art that the scope of the present disclosure is not to be construed as being limited by these Examples.

### [Experimental Examples]

### Experimental Example 1. Method for differentiating human neural stem cells (hNSCs) into Oligodendrocytes

To form oligospheres, hNSCs were cultured in an Oligodendrocyte Progenitor Cells (OPCs) medium for 7 days. The hNSCs were dissociated into single OPCs, and then cultured in an OPCs medium for 5 days. After culturing, the medium was replaced with an oligodendrocyte differentiation medium, and further cultured for 14 days to obtain mature oligodendrocytes.

### Experimental Example 2. Treatment of differentiating oligodendrocytes with extracellular a-Synuclein

An a-Syn conditioned medium (SCM) was treated at a concentration of 1000 ng/mL for 72 hours, and LacZ CM (LZCM), used as a control, was treated at the same volume as aSCM. The experiment was conducted by treating aSCM at oligodendrocyte differentiation day 11, and then matching an end point of the oligodendrocyte differentiation ended and an end point of 72 hours of CM treatment.

### Experimental Example 3. NM-103 treatment in differentiating oligodendrocytes

NM-103 was used at 10 µg/mL and 50 µg/mL and pretreated 30 minutes before CM treatment.

### Experimental Example 4. Immunofluorescence staining

Cells were immobilized with 4% PFA (in PBS) on a coverslip. The immobilized cells were treated with 0.1% Triton X-100 and then washed with PBS. The washed cells were treated with a blocking solution (5% bovine serum albumin and 3% goat serum in PBS) and then washed. The cells were treated with a primary antibody diluted in the blocking solution for 16 hours. The cells treated with the primary antibody were washed. The cells treated with the primary antibody were washed, and the washed cells were treated with a secondary antibody conjugated with a fluorescent dye Alexa488, Cy2, Rhodamine red-X, or Alexa647 for 2 hours. The cells treated with the secondary antibody were washed with PBS. Nuclei were further stained with Hoechst 33342. The coverslips were made as slides using a mounting solution. Thereafter, the slides were observed and analyzed using Zeiss LSM 900 with Airyscan 2 (Zeiss, Oberkochen, Germany).

### Experimental Example 5. Tg mouse animal experiment

aSyn A53T Tg mice (line G2-3) expressing a human aSyn A53T gene under a PrP promoter were used as an MSA disease model. In vivo efficacy evaluation was conducted by administering NM-103 to 6-month-old diseased mice.

### Experimental Example 6. Immunofluorescence staining using brain tissue sections

Sections were obtained from mouse brain tissue fixed with 4% PFA using a Cryotome device. The sections were permeabilized using 0.2% Triton X-100 and a 0.5% NGS (Normal Goat Serum) solution. Thereafter, the sections were washed with PBS and treated with a primary antibody for 16 hours. The sections treated with the primary antibody were washed with PBS, and then treated with a secondary antibody for 2 hours under a light-shielding condition and subjected to immunofluorescence staining. The stained brain tissue was mounted using coverslip and slide and observed using Zeiss LSM 900 with Airyscan 2 (Zeiss, Oberkochen, Germany).

### Experimental Example 7. Analysis of nerve myelin using TEM

Brain tissue was fixed with 4% formaldhyde and 1% glutaraldehyde and stirred at 4°C for 16 hours. After stirring, the brain tissue was post-fixed with 1% osmium tetroxide for 30 minutes. The brain tissue samples were treated with epoxy resin, and then dehydrated with 70%, 80%, 85%, 90%, 95%, and 100% of ethanol solutions. Brain tissue sections were obtained using an ultramicotome, and then sample sections were obtained on Cu grids and stained with uranyl acetate and lead citrate. The prepared tissue was imaged using Cryo-TEM to observe the nerve myelin.

### [Examples]

### Example 1. Construction of In vitro MSA disease cell model using human neural stem cells

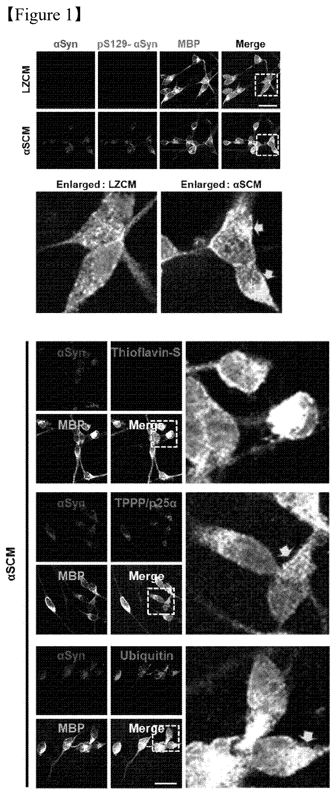

Oligodendrocytes (OLs) were obtained by differentiating human neural stem cells (hNSCs). An in vitro multiple system atrophy (MSA) cell model was constructed by treating the differentiated OLs with aSyn conditioned media (aSCM) containing a large amount of a-synuclein (aSyn), a disease-related toxic protein, for 72 hours. The results of confirming the constructed in vitro MSA cell model were shown in FIG. 1.

As shown in FIG. 1, when the OLs differentiated from hNSCs were treated with aSCM, TPPP/p25a and ubiquitin were detected in the same location as aSyn. The TPPP/p25a ubiquitin was a characteristic of glial cytoplasmic inclusion (GCI) found in MSA patients.

### Example 2. Identification of DNA damage and cellular senescence in In vitro MSA disease cell model

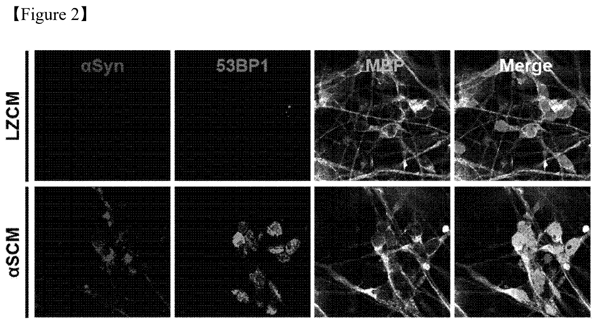

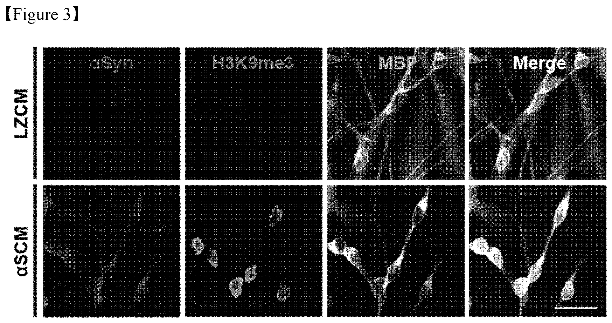

Through immunohistochemical staining, DNA damage and cellular senescence in the in vitro MSA disease cell model constructed in Example 1 were identified. The results of confirming DNA damage in the in vitro MSA disease cell model were shown in FIG. 2, and the results of confirming cellular senescence were shown in FIG. 3.

As shown in FIG. 2, DNA damage associated with degenerative brain diseases was identified in an in vitro MSA cell model induced by aSCM treatment.

As shown in FIG. 3, cellular senescence associated with degenerative brain diseases was identified in the in vitro MSA cell model induced by aSCM treatment.

### Example 3. Construction of in vitro MSA disease cell model treated with NM-103

The in vitro MSA disease cell model constructed in Example 1 above was treated with 50 µg/mL of NM-103 before 30 minutes of treatment with aSCM for 72 hours. The NM-103 was an anti-toll-like receptor 2 (TLR2) antibody, also called Tomaralimab. The specific sequences were as shown in Table 1.

**[Table 1]**

| | Name | | SEQ ID NO: | Sequence |
|---|---|---|---|---|
| | Heavy chain | CDR1 | 1 | TTYGIN |
| | | CDR2 | 2 | GWIYPRDGSTNFNENFKD |
| | | CDR3 | 3 | LTGGTFLDY |
| | Light chain | CDR1 | 4 | RASESVEYYGTSLMQ |
| | | CDR2 | 5 | GASNVES |
| | | CDR3 | 6 | QQSRKLPWT |
| NM-103 | Heavy chain variable region | | 7 | QVQLVQSGSELKKPGASVKLSCKASGFTFTTYGI NWVRQAPGQGLEWIGWIYPRDGSTNFNENFKDR ATITVDTSASTAYMELSSLRSEDTAVYFCARLTG GTFLDYWGQGTTVTVSS |
| | Light chain variable region | | 8 | DIVLTQSPATLSLSPGERATLSCRASESVEYYGTS LMQWYQQKPGQPPKLLIFGASNVESGVPDRFSG SGSGTDFTLKISRVEAEDVGMYFCQQSRKLPWTF GGGTKVEIK |
| | Full sequence of heavy chain | | 9 | QVQLVQSGSELKKPGASVKLSCKASGFTFTTYGI NWVRQAPGQGLEWIGWIYPRDGSTNFNENFKDR ATITVDTSASTAYMELSSLRSEDTAVYFCARLTG GTFLDYWGQGTTVTVSSASTKGPSVFPLAPCSRS TSESTAALGCLVKDYFPEPVTVSWNSGALTSGV HTFPAVLQSSGLYSLSSVVTVPSSSLGTKTYTCN VDHKPSNTKVDKRVESKYGPPCPPCPAPEFLGGP SVFLFPPKPKDTLMISRTPEVTCVVVDVSQEDPE VQFNWYVDGVEVHNAKTKPREEQFNSTYRVVS VLTVLHQDWLNGKEYKCKVSNKGLPSSIEKTISK AKGQPREPQVYTLPPSQEEMTKNQVSLTCLVKG FYPSDIAVEWESNGQPENNYKTTPPVLDSDGSFF LYSRLTVDKSRWQEGNVFSCSVMHEALHNHYT QKSLSLSLGK |
| | Full sequence of light chain | | 10 | DIVLTQSPATLSLSPGERATLSCRASESVEYYGTS LMQWYQQKPGQPPKLLIFGASNVESGVPDRFSG SGSGTDFTLKISRVEAEDVGMYFCQQSRKLPWTF GGGTKVEIKRTVAAPSVFIFPPSDEQLKSGTASVV CLLNNFYPREAKVQWKVDNALQSGNSQESVTE QDSKDSTYSLSSTLTLSKADYEKHKVYACEVTH QGLSSPVTKSFNRGEC |

### Example 4. Evaluation of efficacy of NM-103 in In vitro MSA disease cell model

### 4-4. Reduction of GCI as phenomenon of MSA disease by NM-103

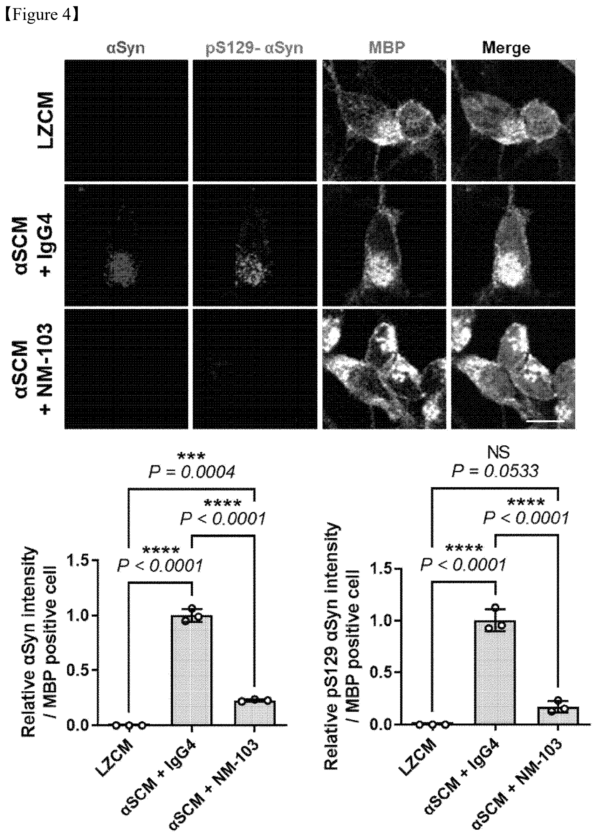

The GCI reduction effect of NM-103 was evaluated in an in vitro MSA disease cell model, and the results were shown in FIG. 4.

As shown in FIG. 4, when an anti-TLR2 antibody NM-103 was treated in the in vitro MSA disease cell model, it was confirmed that serine 129-phosphorylated alpha-synuclein (pS 129-aSyn), a GCI disease marker, was reduced.

### 4-2. Reduction of DNA damage as phenomenon of MSA disease by NM-103

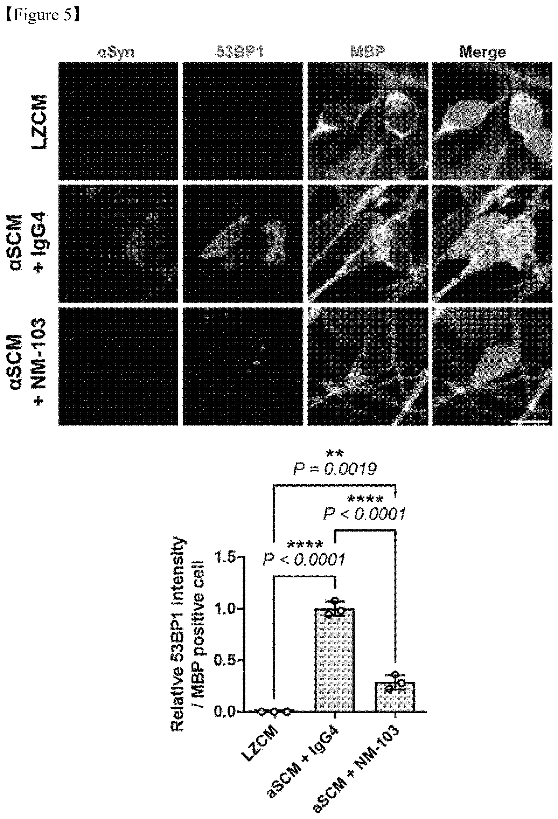

The DNA damage reduction effect of NM-103 was evaluated in an in vitro MSA disease cell model, and the results were shown in FIG. 5.

As shown in FIG. 5, when an anti-TLR2 antibody NM-103 was treated in the in vitro MSA disease cell model, it was confirmed that p53-binding protein 1 (53BP1), a DNA damage marker, was reduced.

### 4-3. Reduction of cellular senescence symptom as phenomenon of MSA disease by NM-103

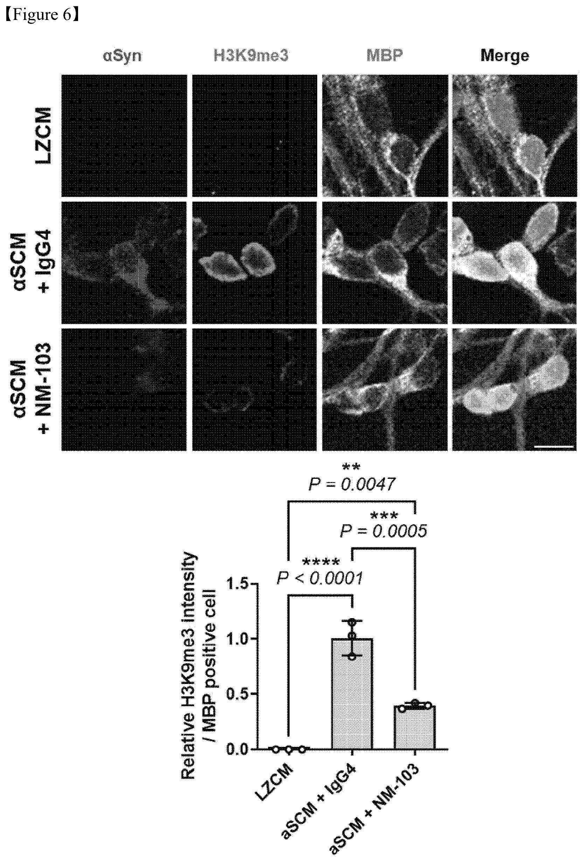

The cellular senescence reduction effect of NM-103 was evaluated in an in vitro MSA disease cell model, and the results were shown in FIG. 6.

As shown in FIG. 6, when an anti-TLR2 antibody NM-103 was treated in the in vitro MSA disease cell model, it was confirmed that H3K9m3 as a cellular senescence marker was reduced.

### Example 5. Therapeutic efficacy experiment of NM-103 in MSA in vivo model using alpha-synuclein disease mutant overexpressing mice

### 5-1. Survival rate according to NM-103 treatment

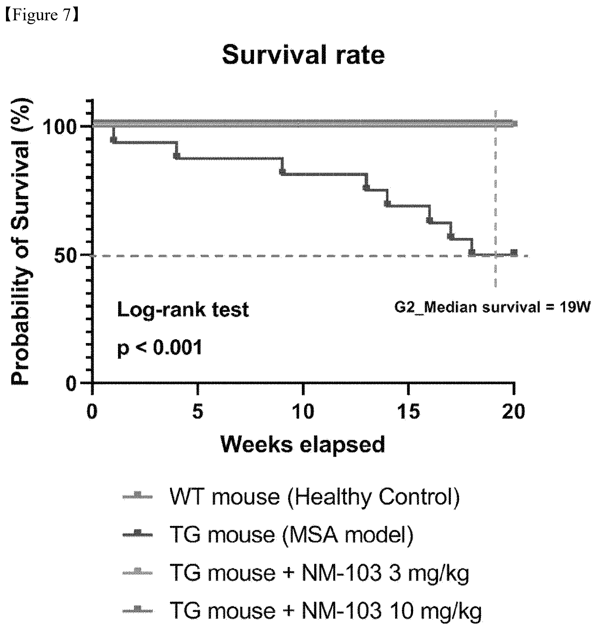

The survival rate of subjects treated with NM-103 was analyzed in a MSA in vivo model. NM-103 was injected into the tail vein (tail vein intravenous (IV) injection). The results of analyzing the survival rate were shown in FIG. 7.

As shown in FIG. 7, in the MSA in vivo model (TG mouse), it was shown that dead subjects were gradually increased over time, and the survival rate was 50%. In contrast, NM-103 administered groups (TG mouse+NM-103 3 mg/kg, TG mouse+NM-103 10 mg/kg) showed the survival rate of 100%.

### 5-2. Motor ability improvement effect according to NM-103 treatment

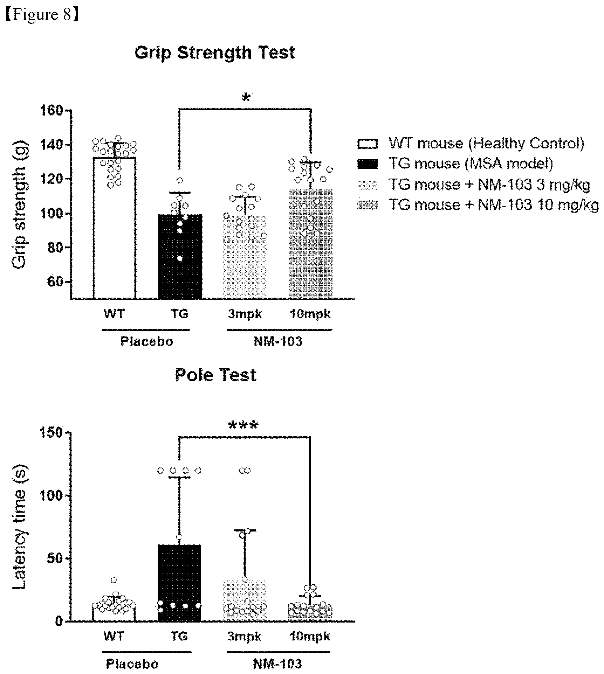

Through GST and Pole Test behavioral tests, a motor ability improvement effect according to treatment of NM-103 was confirmed in the MSA in vivo model. The results of confirming the motor ability improvement effect were shown in FIG. 8.

As shown in FIG. 8, the NM-103 administered groups (TG mouse+NM-103 3 mg/kg, TG mouse+NM-103 10 mg/kg) showed improved motor ability.

### 5-3. Reduction of MSA pathological marker GCI by NM-103

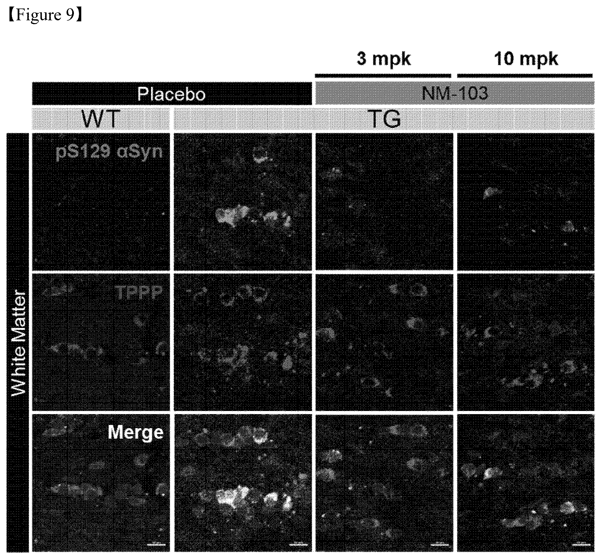

Through fluorescence staining analysis, a MSA marker GCI according to treatment of NM-103 was confirmed in the MSA in vivo model. The results of confirming the expression of GCI were shown in FIG. 9.

As shown in FIG. 9, in the NM-103 administered groups (TG mouse+NM-103 3 mpk, TG mouse+NM-103 10 mpk), it was shown that a MSA disease-specific marker GCI was reduced in Oligodendrocytes.

### 5-4. Reduction of neuroinflammation in MSA mouse model by NM-103

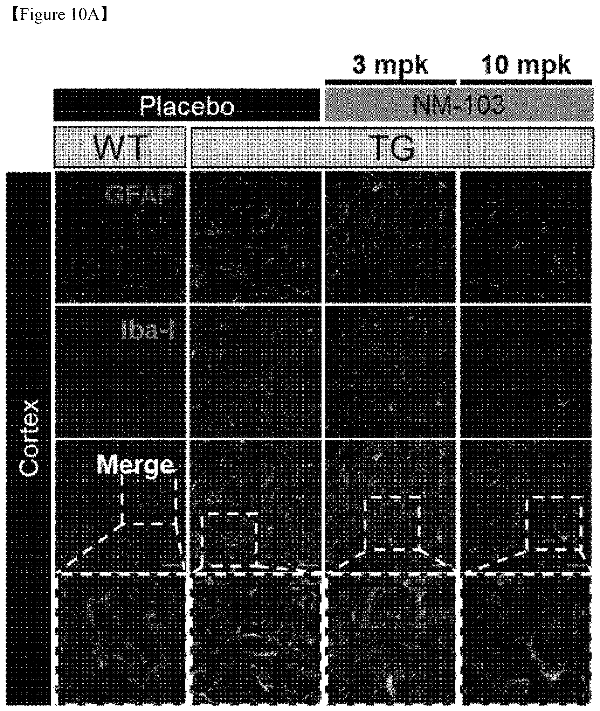

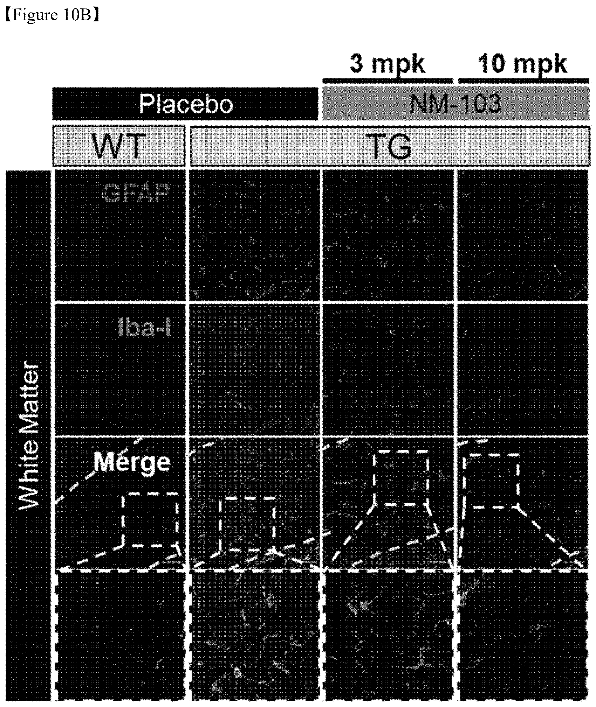

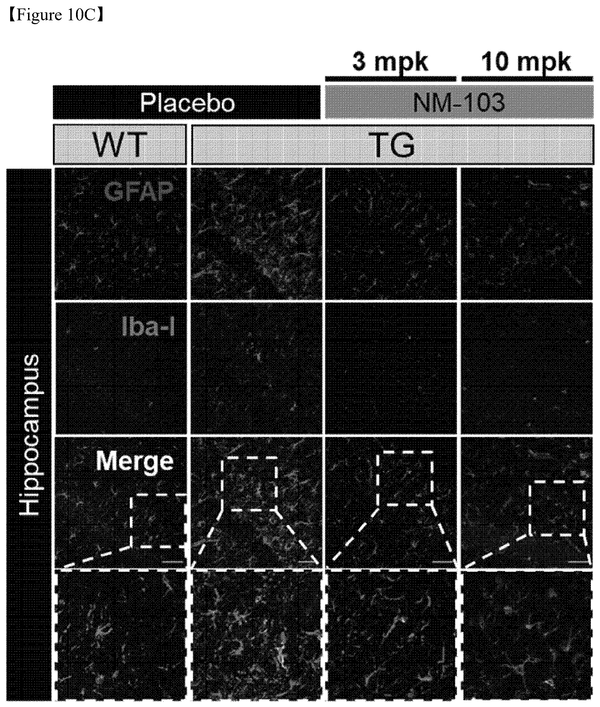

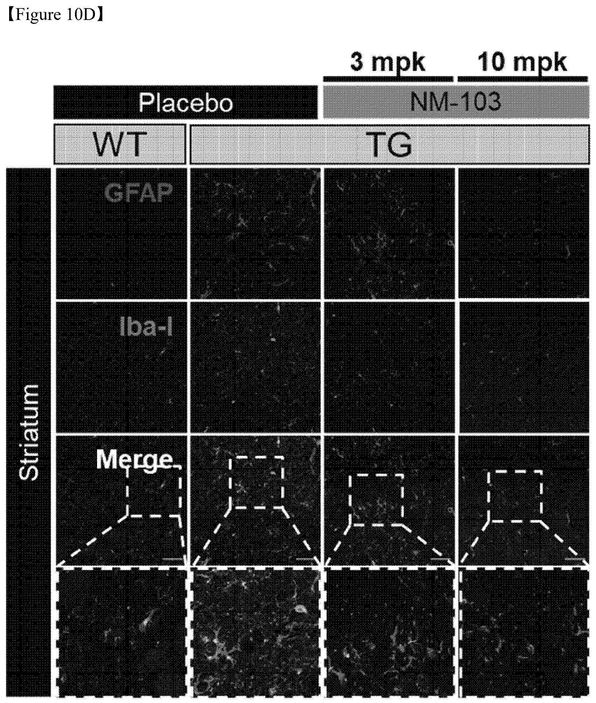

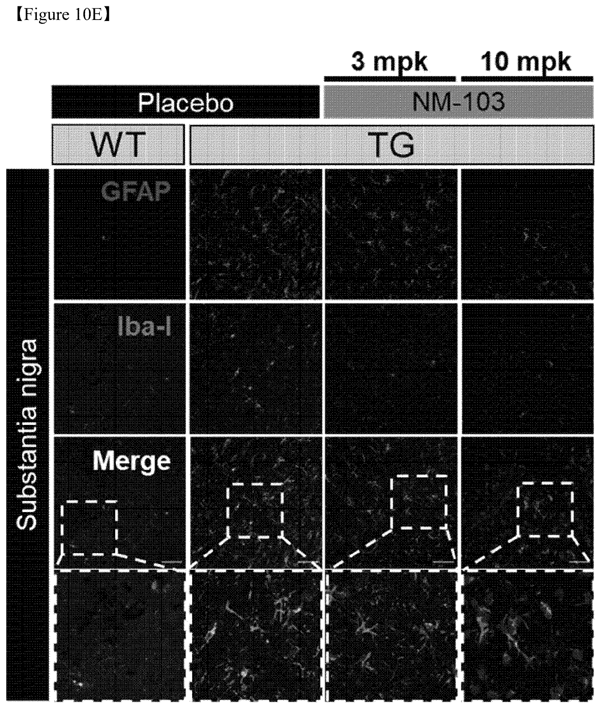

Through fluorescence staining analysis, a neuroinflammation reduction effect according to treatment of NM-103 was confirmed in the MSA in vivo model. The results confirming the neuroinflammation reduction effects of NM-103 in the Cortex, White Matter, Hippocampus, Striatum, and Substantia nigra were shown in FIGS. 10A to 10E, respectively.

As shown in FIGS. 10A to 10E, in the NM-103 administered groups (TG mouse+NM-103 3 mpk, TG mouse+NM-103 10 mpk), it was shown that brain neuroinflammation (GFAP, Iba-I) markers induced by glial cell activity were reduced in various brain regions (Cortex, White Matter, Hippocampus, Striatum, and Substantia nigra).

### 5-6. Reduction of inflammasomes in MSA mouse model by NM-103

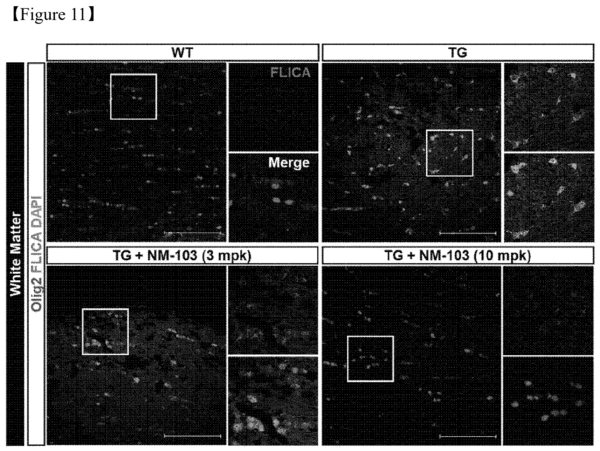

Through FLICA assay, an inflammasome reduction effect according to treatment of NM-103 was confirmed in the MSA in vivo model. The results of confirming the inflammasome reduction effect were shown in FIG. 11.

As shown in FIG. 11, in a MSA model (TG), it was confirmed that inflammasomes were increased in oligodendrocytes. In contrast, the NM-103 administered groups (TG mouse+NM-103 3 mpk, TG mouse+NM-103 10 mpk) showed reduced inflammasomes.

### 5-7. Neurofibril recovery in MSA mouse model by NM-103

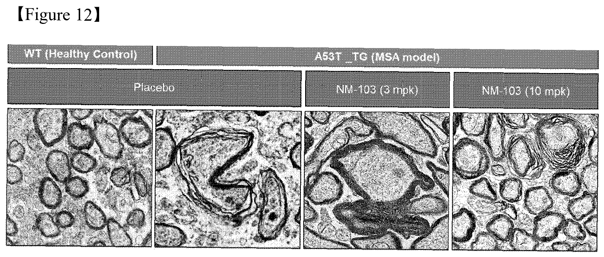

A neurofibril recovery effect according to treatment of NM-103 was confirmed in the MSA in vivo model. The results of confirming the neurofibril recovery effect were shown in FIG. 12.

As shown in FIG. 12, in the MSA model (TG), it was confirmed that nerve myelin damage and demyelination were increased. In contrast, in the NM-103 administered groups (TG mouse+NM-103 3 mpk, TG mouse+NM-103 10 mpk), it was confirmed that the nerve myelin was protected and the demyelination was suppressed (i.e., neurofibril recovery).

As described above, specific parts of the present disclosure have been described in detail, and it will be apparent to those skilled in the art that these specific techniques are merely preferred embodiments, and the scope of the present disclosure is not limited thereto. Therefore, the substantial scope of the present disclosure will be defined by the appended claims and their equivalents.

## Claims

1. A pharmaceutical composition for preventing or treating multiple system atrophy, comprising an antibody or an antigen binding fragment thereof that specifically binds to toll-like receptor 2 (TLR2) comprising:
a heavy chain variable region including a heavy chain CDR1 including an amino acid sequence represented by SEQ ID NO: 1,
a heavy chain CDR2 including an amino acid sequence represented by SEQ ID NO: 2, and
a heavy chain CDR3 including an amino acid sequence represented by SEQ ID NO: 3; and
a light chain variable region including a light chain CDR1 including an amino acid sequence represented by SEQ ID NO: 4,
a light chain CDR2 including an amino acid sequence represented by SEQ ID NO: 5, and
a light chain CDR3 including an amino acid sequence represented by SEQ ID NO: 6.

2. The composition of claim 1, wherein the antibody that specifically binds to TLR2 comprises a heavy chain variable region represented by an amino acid sequence represented by SEQ ID NO: 7.

3. The composition of claim 1, wherein the antibody that specifically binds to TLR2 comprises a light chain variable region represented by an amino acid sequence represented by SEQ ID NO: 8.

4. The composition of claim 1, wherein the antibody that specifically binds to TLR2 comprises
a heavy chain represented by an amino acid sequence represented by SEQ ID NO: 9; and
a light chain represented by an amino acid sequence represented by SEQ ID NO: 10.

5. A veterinary composition for preventing or treating multiple system atrophy, comprising an antibody or an antigen binding fragment thereof that specifically binds to toll-like receptor 2 (TLR2) comprising:
a heavy chain variable region including a heavy chain CDR1 including an amino acid sequence represented by SEQ ID NO: 1,
a heavy chain CDR2 including an amino acid sequence represented by SEQ ID NO: 2, and
a heavy chain CDR3 including an amino acid sequence represented by SEQ ID NO: 3; and
a light chain variable region including a light chain CDR1 including an amino acid sequence represented by SEQ ID NO: 4,
a light chain CDR2 including an amino acid sequence represented by SEQ ID NO: 5, and
a light chain CDR3 including an amino acid sequence represented by SEQ ID NO: 6.

6. A method for treating multiple system atrophy comprising:
administering, to a subject, an effective amount of an antibody or an antigen binding fragment thereof that specifically binds to toll-like receptor 2 (TLR2), comprising a heavy chain variable region including a heavy chain CDR1 including an amino acid sequence represented by SEQ ID NO: 1,
a heavy chain CDR2 including an amino acid sequence represented by SEQ ID NO: 2, and
a heavy chain CDR3 including an amino acid sequence represented by SEQ ID NO: 3; and
a light chain variable region including a light chain CDR1 including an amino acid sequence represented by SEQ ID NO: 4,
a light chain CDR2 including an amino acid sequence represented by SEQ ID NO: 5, and
a light chain CDR3 including an amino acid sequence represented by SEQ ID NO: 6.
